# FASCICULE DE BREVET EUROPEEN

(11) **EP 0 662 987 B1**
(45) Date de publication et mention de la délivrance du brevet: **11.03.1998**
(21) Numéro de dépôt: 94923743.2
(22) Date de dépôt: 27.07.1994
(51) Int. Cl.: C08L 5/00, C08J 3/09, A61K 7/06, A61K 7/48

(54) **PROCEDE POUR LA SOLUBILISATION DU POLY(1-3)GLUCOPYRANOSE**
VERFAHREN ZUR SOLUBILISIERUNG VON POLY(1-3)GLUKOPYRANOSE
METHOD FOR SULUBILISING POLY(1-3)GLUCOPYRANOSE

(30) Priorité: 28.07.1993 FR 9309389
(43) Date de publication de la demande: 19.07.1995
(73) Titulaire: INSTITUT DE RECHERCHES ET D'INNOVATIONS SCIENTIFIQUES (I.R.I.S.), F-75016 Paris (FR)
(72) Inventeur: FRITSCH, Marie, Claire, F-75007 Paris (FR); PEYRAMAURE, Pierre, F-92150 Suresnes (FR)
(74) Mandataire: de Saint-Palais, Arnaud Marie
(86) Numéro de dépôt international: FR9400940
(87) Numéro de publication internationale: WO9504107

(56) Documents cités:
- US-A- 3 659 025

## Description

La présente invention concerne un procédé pour la solubilisation du poly(l-3)glucopyranose dans un milieu compatible à une incorporation ultérieure dans une préparation cosmétique. Elle a également pour objet une composition cosmétique utilisant du poly(1-3)glucopyranose solubilisé par ledit procédé.

On rappelle que le poly(1-3)glucopyranose est un polysaccharide à longue chaîne extrait par exemple de parois cellulaires de levures, et qui se présente sous la forme d'une poudre de couleur beige, inodore et insoluble dans l'eau, et est commercialisé par la firme "Immudyne Inc" sous la marque "Nayad". Il se classe dans la catégorie chimique des polyosides homogènes, catégorie qui regroupe entre autres :
- les dextranes qui sont solubles en milieu aqueux, formant des solutions colloïdales de viscosité variable en fonction du poids moléculaire,
- l'amidon qui n'est pas soluble dans l'eau mais qui, porté à 100°C, forme l'empois bien connu,
- la cellulose qui est insoluble dans l'eau et dans les solvants usuels.

Les études conduites jusqu'ici sur la solubilisation des polysaccharides autres que les amidons font apparaître que :
. Les polysaccharides neutres tels que dextrane, levane, scléroglucane peuvent être solubilisés directement par le DMSO ((CH₃)₂SO). Toutefois, ce solvant présente l'inconvénient d'être incompatible avec l'incorporation dans un produit cosmétique.
. L'action combinée de la 4-méthylmorpholine-n-oxyde et du DMSO permet une solubilisation globale des polysaccharides autres que l'amidon. Toutefois, dans ce cas, une étape de chauffage à 120°C est nécessaire à l'extraction des produits. De ce fait, l'intégrité du poly(l-3)glucopyranose ne peut pas être garantie et ce procédé est en outre sujet aux mêmes problèmes d'incompatibilité que précédemment.
. L'utilisation de l'hydroxyde de sodium NaOH qui, pourtant, engendre des variations de viscosité du scléroglucane, n'a pas permis d'obtenir de résultat significatif dans le cas du poly(1-3)glucopyranose : ces observations tendent à confirmer que le poly(1-3)glucopyranose n'est pas véritablement un scléroglucane.

Ces études n'ont donc pas permis de déterminer un procédé de solubilisation applicable au poly(1-3)glucopyranose et qui soit compatible avec des applications cosmétiques.

La Demanderesse a donc effectué des essais en utilisant des agents tels que des polyalcools pour mouiller la poudre et faciliter sa dispersion dans l'eau.

A ce titre, une série d'expériences ont été effectuées en vue d'évaluer la solubilité du poly(1-3)glucopyranose dans une gamme de mélanges sorbitol/eau distillée, à des températures allant de +25°C à +100°C, afin de sélectionner le solvant permettant une dissolution optimale du produit à la température la plus basse possible.

Au cours de ces expériences, les solutions ont été soumises à des températures croissantes (de 10°C en 10°C jusqu'à 100°C) pendant des périodes d'une à deux heures, avec des temps de repos d'environ une 1/2 heure entre chaque pallier. Dès que l'aspect des solutions l'a permis (homogénéité et absence d'agrégats en suspension), une lecture spectrophotométrique à 600 nm a été effectuée afin d'évaluer le degré d'opacité.

L'ensemble des solutions a été maintenu à une température ambiante pendant 48 heures. A l'issue de ce délai, l'aspect et l'absorbance à 600 nm ont été enregistrés.

Les résultats obtenus ont été indiqués sur le Tableau I ci-après qui ne prend en compte les températures qu'à partir de 50°C (la mesure d'absorbance n'ayant pu être effectuée en deçà de cette température, la majorité des solutions n'étant pas homogène).

Ils montrent que quelle que soit la composition du mélange solvant, une étape de chauffage est indispensable à la dispersion du principe actif, la température minimale étant de 50°C. Par ailleurs, plus la proportion de sorbitol augmente, plus la dispersion est aisée à basse température, et plus l'opacité du mélange diminue. En outre, le seuil de température nécessaire à l'obtention d'une apparente dissolution du principe actif diminue d'autant plus que la proportion de sorbitol augmente.

**TABLEAU I**

| Composition du mélange solvant | | TEMPERATURE | | | | | |
|---|---|---|---|---|---|---|---|
| | | +50°C | | +60°C | | +80°C | +100°C |
| Eau (V) | Sorbitol (V) | 1 h | 2 h | 1 h | 2 h | 1 h | 1 h |
| 100 | 0 | 1,368 | 1,419 | 1,387 | 1,361 | 1,299 | 1,369 |
| 90 | 10 | 1,347 | 1,351 | 1,335 | 1,310 | 1,293 | 1,292 |
| 80 | 20 | 1,259 | 1,255 | 1,240 | 1,246 | 1,234 | 1,206 |
| 70 | 30 | 1,150 | 1,142 | 1,110 | 1,096 | 1,080 | 1,098 |
| 60 | 40 | 1,035 | 1,029 | 1,002 | 0,997 | 0,975 | 0,996 |
| 50 | 50 | 0,912 | 0,888 | 0,849 | 0,822 | 0,817 | 0,838 |
| 40 | 60 | 0,774 | 0,774 | 0,736 | 0,730 | 0,704 | 0,720 |
| 30 | 70 | 0,784 | 0,716 | 0,668 | 0,653 | 0,609 | 0,633 |
| 20 | 80 | 0,637 | 0,594 | 0,562 | 0,551 | 0,512 | 0,535 |
| 10 | 90 | 0,504 | 0,492 | 0,482 | 0,467 | 0,438 | 0,449 |
| 0 | 100 | 0,477 | 0,433 | 0,418 | 0,420 | 0,377 | 0,394 |

A partir de 80% v/v de sorbitol, jusqu'à 48 heures de repos, la solution reste stable à température ambiante puis fait l'objet d'un dépôt qui peut disparaître avec l'agitation de la solution. Elle est instable à 0°C et à 50°C.

Il est clair que même si ces résultats sont encourageants et constituent un réel progrès, en particulier pour des pourcentages en volume de sorbitol de l'ordre de 90%, les instabilités constatées au bout de 48 heures et à 0°C et 50°C demeurent très gênantes.

L'invention a donc plus particulièrement pour but de supprimer ces inconvénients.

Elle propose à cet effet, un procédé de solubilisation du poly(1-3)glucopyranose qui consiste à le mélanger à une composition comprenant du sorbitol dans une proportion supérieure à 80% en poids/poids, de préférence à 90% en poids/poids de la composition), et d'un additif comprenant deux composés choisis parmi les trois composés suivants : l'eau, la glycérine et le propylène glycol.

Dans le cas où l'additif comprend de l'eau, sa composition sera de préférence :
- eau : 2,0% à 4,5% en poids/poids du mélange total
- glycérine ou propylène glycol : 7,4% à 4,9% en poids/poids du mélange total

Dans le cas où l'additif ne comprend pas d'eau, sa composition sera, de préférence :
- glycérine : 2,4% à 7,4% en poids/poids du mélange total
- propylène glycol : 7,0% à 2,0% en poids/poids du mélange total (solution qui donne les meilleurs résultats en terme de stabilité)

Avantageusement, l'additif représentera 9,4% en poids/poids du mélange.

Des essais effectués sur un mélange comprenant du poly(1-3)glucopyranose dans une proportion de 0,1% en poids/poids du mélange total, et diverses compositions comprenant du sorbitol, du phénonip ainsi que de l'eau déminéralisée et/ou du propylène glycol, ont permis d'établir le Tableau II de résultats ci-après :

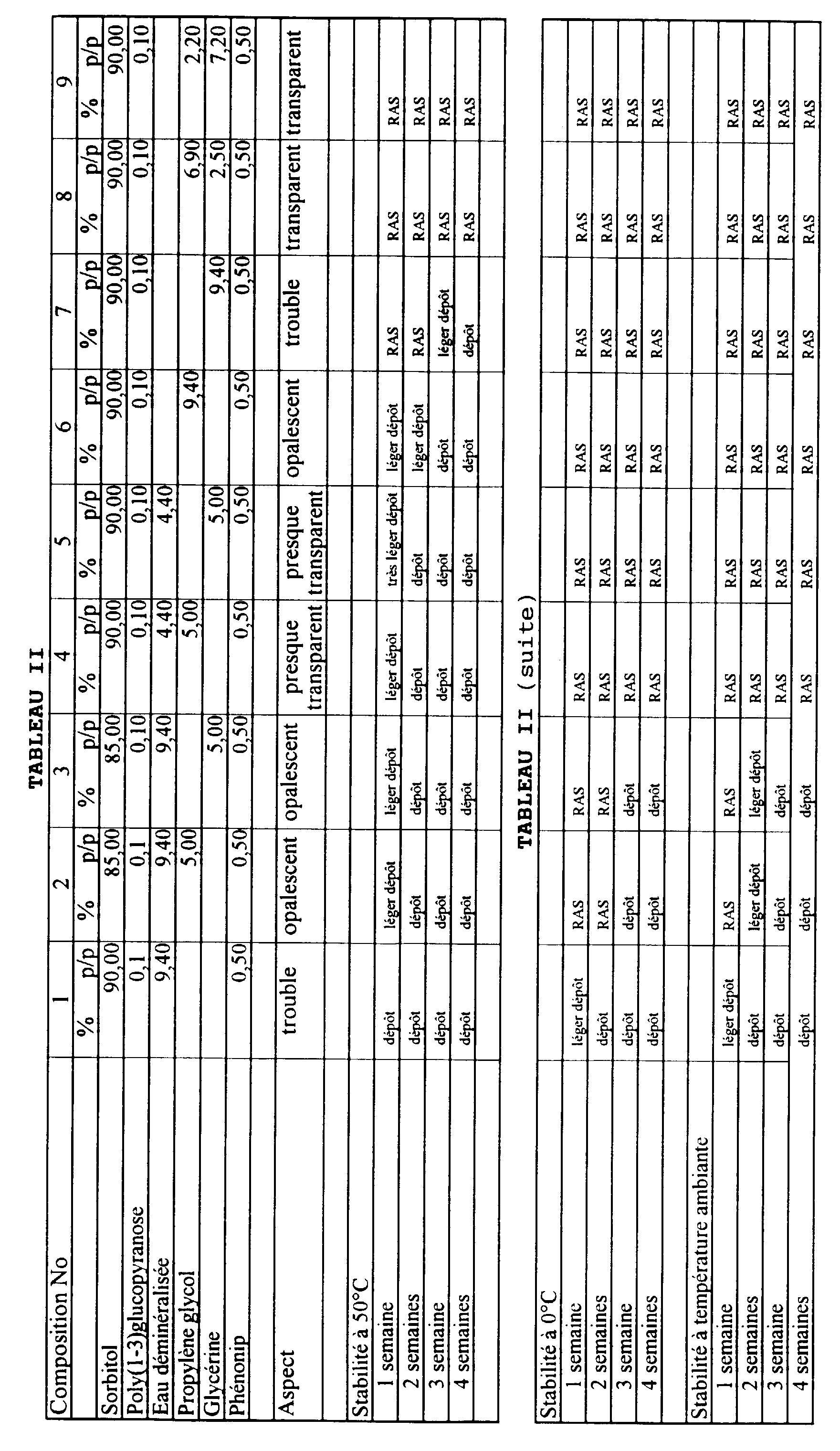

Dans ce tableau, la colonne 1 est affectée à un essai de solubilisation faisant intervenir du sorbitol (90% en poids/poids), de l'eau déminéralisée (9,40% en poids/poids) et un adjuvant conservateur tel que du phénonip (0,5% en poids/poids). Cet essai ne fait que confirmer les résultats précédemment indiqués à savoir :
- amélioration de la dispersion,
- amélioration de l'aspect (pseudo-solution moins trouble),
- stabilité pendant 48 heures à température ambiante puis formation d'un dépôt qui peut disparaître temporairement avec l'agitation de la solution,
- instabilité à 50°C et à 0°C.

Les colonnes 2 et 3 correspondent à des essais dans lesquels une partie du sorbitol a été remplacée par du propylène glycol et de la glycérine. Les résultats qui ont été constatés à la suite de ces essais sont :
- amélioration de l'aspect (solution opalescente),
- amélioration de la stabilité à la température ambiante et à 0°C, (néanmoins, cette stabilité demeure insuffisante),
- pas d'amélioration de la stabilité à 50°C.

Les résultats indiqués dans les colonnes 4 et 5 sont obtenus à l'aide d'un mélange de propylène glycol et de glycérine en substitution d'une partie d'eau distillée. Ils font apparaître :
- une amélioration plus importante de l'aspect (presque transparent),
- une stabilité satisfaisante à température ambiante et à 0°C,
- une très légère amélioration de la stabilité à 50°C.

Les colonnes 6 à 9 sont affectées à des compositions dans lesquelles l'eau a été totalement éliminée et remplacée par le propylène glycol ou la glycérine (colonnes 6 et 7) et par une combinaison de propylène glycol et de glycérine en proportions déterminées (colonnes 8 et 9).

Les essais illustrés dans les colonnes 6 et 7 font apparaître les résultats suivants :
- aucune amélioration de l'aspect et un retour à des solutions troubles (glycérine) ou opalescentes (propylène glycol),
- stabilité satisfaisante à température ambiante et à 0°C,
- légère amélioration de la stabilité à 50°C.

Les colonnes 8 et 9 font, par contre, apparaître des résultats très satisfaisants, avec :
- amélioration de l'aspect, solutions transparentes,
- stabilité satisfaisante à température ambiante et à 50°C.

Grâce à ces résultats, il devient possible d'obtenir une solubilisation du poly(l-3)glucopyranose à l'aide de constituants totalement compatibles avec des applications dans le domaine de la cosmétique.

## Revendications

1. Procédé pour la solubilisation du poly(1-3)glucopyranose dans un mélange solvant,
caractérisé en ce que le mélange solvant consiste en une composition comprenant du sorbitol, dans une proportion supérieure à 80% en poids/poids du mélange total, et un additif comprenant deux composés choisis parmi les trois composés suivants : l'eau, la glycérine et le propylène glycol.

2. Procédé selon la revendication 1,
caractérisé en ce que la proportion de sorbitol est égale à 90% en poids/poids du mélange total.

3. Procédé selon la revendication 1,
caractérisé en ce que la proportion du glucopyranose est de l'ordre de 0,1% en poids/poids du mélange total.

4. Procédé selon l'une des revendications 1 et 2,
caractérisé en ce que dans le cas où l'additif comprend de l'eau, sa composition est :
- eau : 2,0% à 4,5% en poids/poids du mélange total
- glycérine ou propylène glycol : 7,4% à 4,9% en poids/poids du mélange total

5. Procédé selon la revendication 1,
caractérisé en ce que dans le cas où l'additif ne comprend pas d'eau, sa composition est :
- glycérine : 2,4% à 7,4% en poids/poids du mélange total
- propylène glycol : 7,0% à 2,0% en poids/poids du mélange total

6. Procédé selon la revendication 1,
caractérisé en ce que l'additif représente 9,4% en poids/poids du mélange.

7. Procédé selon la revendication 1,
caractérisé en ce que l'additif comprend en outre un adjuvant conservateur dans une proportion de 0,5% en poids/poids du mélange total.

8. Procédé selon la revendication 7,
caractérisé en ce que l'adjuvant conservateur est du phénonip.

9. Composition cosmétique utilisant du poly(1-3)glucopyranose solubilisé par le procédé selon la revendication 1.

## Patentansprüche

1. Verfahren zur Solubilisierung von Poly(1-3) glucopyranose in einer Lösungsmittelmischung,
dadurch gekennzeichnet, dass die Lösungsmittelmischung aus einer Zusammensetzung besteht, die Sorbitol in einem Anteil von mehr als 80% der Gesamtmischung und ein Additiv enthält, das zwei Verbindungen enthält, die aus den drei folgenden Verbindungen ausgewählt sind : Wasser, Glycerin und Propylengycol.

2. Verfahren nach Anspruch 1,
dadurch gekennzeichnet, dass der Anteil von Sorbitol gleich 90% der Gesamtmischung ist.

3. Verfahren nach Anspruch 1,
dadurch gekennzeichnet, dass der Anteil von Glucopyranose etwa 0,1% der Gesamtmischung ausmacht.

4. Verfahren nach einem der Ansprüche 1 und 2,
dadurch gekennzeichnet, dass, wenn das Additiv Wasser enthält, seine Zusammensetzung die folgende ist :
- Wasser : 2,0% bis 4,5% der Gesamtmischung,
- Glycerin oder Propylenglycol : 7,4% bis 4,9% der Gesamtmischung.

5. Verfahren nach Anspruch 1,
dadurch gekennzeichnet, dass, wenn das Additiv kein Wasser enthält, seine Zusammensetzung die folgende ist :
- Glycerin : 2,4% bis 7,4% der Gesamtmischung,
- Propylenglycol : 7,0% bis 2,0% der Gesamtmischung.

6. Verfahren nach Anspruch 1,
dadurch gekennzeichnet, dass das Additiv 9,4% der Mischung ausmacht.

7. Verfahren nach Anspruch 1,
dadurch gekennzeichnet, dass das Additiv ausserdem einen konservierenden Zusatzstoff in einem Anteil von 0,5% der Gesamtmischung enthält.

8. Verfahren nach Anspruch 7,
dadurch gekennzeichnet, dass der konservierende Zusatzstoff Phenonip ist.

9. Kosmetische Zusammensetzung, die durch das Verfahren gemäss Anspruch 1 gelöstes Poly(1-3)glucopyranose verwendet.

## Claims

1. A method for solubilizing poly(1-3) glucopyranose in a solvent mixture,
characterized in that the solvent mixture consists of a composition comprising sorbitol, in a proportion exceeding 80% by weight of the total mixture and an additive comprising two compounds selected from among the following three compounds : water, glycerin and propylene glycol.

2. The method as claimed in claim 1,
characterized in that the proportion of sorbitol is equal to 90% by weight of the total mixture.

3. The method as claimed in claim 1,
characterized in that the proportion of glucopyranose is of the order of 0.1% by weight of the total mixture.

4. The method as claimed in one of claims 1 and 2,
characterized in that when the additive comprises water, its composition is :
- water 2.0% to 4.5% by weight of the total mixture,
- glycerin or propylene glycol : 7.4% to 4.9% by weight of the total mixture.

5. The method as claimed in claim 1,
characterized in that when the additive does not comprise water, its composition is :
- glycerin : 2.4% to 7.4% by weight of the total mixture,
- propylene glycol : 7.0% to 2.0% by weight of the total mixture.

6. The method as claimed in claim 1,
characterized in that the additive represents 9.4% by weight of the mixture.

7. The method as claimed in claim 1,
characterized in that the additive further comprises a preservative in a proportion of 0.5% by weight of the total mixture.

8. The method as claimed in claim 7,
characterized in that the preservative is phenonip.

9. A cosmetic composition using poly(1-3)glucopyranose solubilized by the method as claimed in claim 1.
